# EUROPÄISCHE PATENTANMELDUNG

(11) **EP 1 304 378 A1**
(43) Veröffentlichungstag der Anmeldung: **23.04.2003**
(21) Anmeldenummer: 02090305.0
(22) Anmeldetag: 27.08.2002
(51) Int. Cl.: C12N 15/12, C12N 15/11, C12N 9/04, C12N 5/10, C12N 1/21, C12Q 1/68, G01N 33/50, G01N 33/68, A61K 38/17, A61K 31/7088, A61K 39/395

(54) **ARL-2 für die Fertilitätskontrolle**

(30) Priorität: 19.10.2001 DE 10152598
(71) Anmelder: Schering Aktiengesellschaft, 13353 Berlin (DE)
(72) Erfinder: Lessl, Monika, 16548 Glienicke-Nordbahn (DE); Peter-Kottig, Michaele, 10437 Berlin (DE)

(57) **Zusammenfassung**

Es wird ein humanes ARL-2 Protein sowie dessen Verwendung für Fertilitätsstörungen, Therapie und Diagnose beschrieben.

SEQUENZPROTOKOLL

<110> Schering AG

<120> ARL-2 und Kontrazeption

<130> 52215

<140>
<141>

<160> 2

<170> PatentIn Ver. 2.1

<210> 1
<211> 1315
<212> DNA
<213> Homo sapiens

<400> 1
agcaggacgt gagacttcta cctgctcact cagaatcatt tccgcaccaa ccatggccac 60
gtttgtggag ctcagtacaa aagccaagat gcccattgtg ggcctgggca cttggaagtc 120
tcttctcggc aaagtgaaag aagcggtgaa ggtggccatt gatgcagaat atcgccacat 180
tgactgtgcc tatttctatg agaatcaaca tgaggtggga gaagccatcc aagagaagat 240
ccaagagaag gctgtgatgc gggaggacct gttcatcgtc agcaagttgt ggcccacttt 300
ctttgagaga ccccttgtga ggaaagcctt tgagaagacc ctcaaggacc tgaagctgag 360
ctatctggac gtctatctta ttcactggcc acagggattc aagactgggg atgacttttt 420
ccccaaagat gataaaggta atatgatcag tggaaaagga acgttcttgg atgcctggga 480
ggccatggag gagctggtgg acgaggggct ggtgaaagcc cttggggtct caaatttcaa 540
ccacttccag atcgagaggc tcttgaacaa acctggactg aaatataaac cagtgactaa 600
ccaggttgag tgtcacccat acctcacgca ggagaaactg atccagtact gccactccaa 660
gggcatcacc gttacggcct acagccccct gggctctccg gatagacctt gggccaaacc 720
tgaggaccct tccctgctgg aggatcccaa gattaaggag attgctgcaa agcacaaaaa 780
aaccacagcc caggttctga tccgtttcca tatccagagg aatgtgacag tgatccccaa 840
gtctgtgaca ccagcacaca ttgttgagaa cattcaggtc tttgacttta aattgagtga 900
tgaggagatg gcaaccatac tcagcttcaa cagaaactgg agggcctttg acttcaagga 960
atcctctcat ttggaggact ttcccttcga tgcagaatat tgaggttgaa tctcctggtg 1020
agattacaca ggggattctc tttcttcgct gaagtgtgac tgtctccact caagaactat 1080
tttagccaag cttatctgag atcacagtga actttgtcct gttgtagacc agaatggagg 1140
tgctgtttta gacatgtatt tctgtatgtt caactaggat aagaatatca cagaaaagca 1200
tggcctgaat aagcaaatga caattttttc cacttatctg atctgatcaa atgtctgtta 1260
agcaccagaa actctgccaa cactgaggat gtaaagataa ataataaaaa ataat 1315

<210> 2
<211> 316
<212> PRT
<213> Homo sapiens

<400> 2
Met Ala Thr Phe Val Glu Leu Ser Thr Lys Ala Lys Met Pro Ile Val
1               5                   10                  15

Gly Leu Gly Thr Trp Lys Ser Leu Leu Gly Lys Val Lys Glu Ala Val
            20                  25                  30

Lys Val Ala Ile Asp Ala Glu Tyr Arg His Ile Asp Cys Ala Tyr Phe
        35                  40                  45

Tyr Glu Asn Gln His Glu Val Gly Glu Ala Ile Gln Glu Lys Ile Gln
    50                  55                  60

Glu Lys Ala Val Met Arg Glu Asp Leu Phe Ile Val Ser Lys Leu Trp
65                  70                  75                  80

Pro Thr Phe Phe Glu Arg Pro Leu Val Arg Lys Ala Phe Glu Lys Thr
                85                  90                  95

Leu Lys Asp Leu Lys Leu Ser Tyr Leu Asp Val Tyr Leu Ile His Trp
            100                 105                 110

Pro Gln Gly Phe Lys Thr Gly Asp Asp Phe Phe Pro Lys Asp Asp Lys
        115                 120                 125

Gly Asn Met Ile Ser Gly Lys Gly Thr Phe Leu Asp Ala Trp Glu Ala
    130                 135                 140

Met Glu Glu Leu Val Asp Glu Gly Leu Val Lys Ala Leu Gly Val Ser
145                 150                 155                 160

Asn Phe Asn His Phe Gln Ile Glu Arg Leu Leu Asn Lys Pro Gly Leu
                165                 170                 175

Lys Tyr Lys Pro Val Thr Asn Gln Val Glu Cys His Pro Tyr Leu Thr
            180                 185                 190

Gln Glu Lys Leu Ile Gln Tyr Cys His Ser Lys Gly Ile Thr Val Thr
        195                 200                 205

Ala Tyr Ser Pro Leu Gly Ser Pro Asp Arg Pro Trp Ala Lys Pro Glu
    210                 215                 220

Asp Pro Ser Leu Leu Glu Asp Pro Lys Ile Lys Glu Ile Ala Ala Lys
225                 230                 235                 240

His Lys Lys Thr Thr Ala Gln Val Leu Ile Arg Phe His Ile Gln Arg
                245                 250                 255

Asn Val Thr Val Ile Pro Lys Ser Val Thr Pro Ala His Ile Val Glu
            260                 265                 270

Asn Ile Gln Val Phe Asp Phe Lys Leu Ser Asp Glu Glu Met Ala Thr
        275                 280                 285

Ile Leu Ser Phe Asn Arg Asn Trp Arg Ala Phe Asp Phe Lys Glu Ser
    290                 295                 300

Ser His Leu Glu Asp Phe Pro Phe Asp Ala Glu Tyr
305                 310                 315

## Beschreibung

Die Erfindung betrifft ARL-2 und dessen Verwendung als Arzneimittel für die Beeinflußung von apoptotischen Prozessen, die eine grundlegende Rolle spielen bei der Fertilitätskontrolle.

Zur Kontrolle der Fertilität ist die "klassische Pille" als weibliches Kontrazeptivum, das am häufigsten eingesetzte Mittel der Wahl. Ihre regelmäßige Einnahme führt zur Ovulationshemmung bei der Frau. Das Prinzip dieser Methode ist, dass es durch die Einnahme der Pille zu einer Unterdrückung der endogenen Steroidhormonproduktion im Ovar und somit zu einer Ovulationshemmung kommt. Ein Nachteil ist, dass ein natürlicher Zyklus somit bei der Frau nicht mehr vorhanden ist. Zudem können in Zusammenhang mit der Einnahme der Pille bei Patientinnen mit Risikopotential Nebenwirkungen wie beispielsweise Brustspannungen, Gewichtszunahmen etc. auftreten.

Durch zahlreiche Untersuchungen ist belegt, dass bei der Frau mit zunehmendem Alter die Fertilität abnimmt. Dies ist unter anderem auf eine schlechtere Qualität der Eizelle, eine erhöhte Abortrate, verstärkte Exposition mit Infektionskeimen (z.B. Chlamydien oder Gonokokken) zurückzuführen. Da sich in den Industrieländern jedoch das Alter der Erstgebährenden immer weiter nach hinten verschiebt, ist es notwendig, Möglichkeiten zur Verbesserung der Fertilität zu finden. Dieses trifft für die Frau und auch für den Mann zu. Für Paare mit Fertilitätsstörungen stehen heute die Techniken der assistierten Reproduktion zur Verfügung (*in vitro* Fertilisation (IVF); Gamete intrafallopian transfer (GIFT), Intrauterine Insemination). Diese Methoden sind jedoch invasiv und in den meisten Fällen mit einer vorherigen Stimulation der Follikelreifung bei der Frau durch Proteohormone (Follikel-stimulierendes Hormon/FSH) verbunden. Diese kann zu unangenehmen Nebenwirkungen wie Kopfschmerzen oder Schmerzen im Abdomen, im schlimmsten Fall jedoch auch zum sogenannten ovariellen Hyperstimulationssyndrom führen.

Es besteht die dringende Notwendigkeit neue Substanzen und Mittel zur Fertilitätskontrolle, das heißt sowohl zur Fertilitätsförderung als auch zur Fertilitätshemmung, bereitzustellen.

Fertilitätsstörungen werden häufig durch apoptotische Prozesse verursacht und können durch Einsatz anti-apoptotischer Proteine therapiert werden. Eine Beteiligung der für die Fertilität wichtigen Enzyme LHOP (**I**uteinisierendes **H**ormon abhängiges **O**var **P**rotein) und ARL-1, dem humanen Homolog von LHOP, an der Vermeidung der Apoptose konnte nicht belegt werden. Allerdings war bisher nicht bekannt, ob neben dem ARL-1 weitere humane Homologe existieren, die eine antiapoptotische Funktion besitzen.

Diese Frage wird durch die vorliegende Erfindung gelöst durch die Bereitstellung einer Nukleinsäure umfassend
a. die in Seq ID NO 1 dargestellte Nukleotidsequenz,
b. eine einer Sequenz aus a. im Rahmen der Degeneration des genetischen Codes entsprechende Nukleotidsequenz oder
c. eine mit den Sequenzen aus a. oder b, unter stringenten Bedingungen hybridisierende Nukleotidsequenz mit der Funktion eines humanen ARL-2 Proteins.

Der Begriff "Hybridisierung unter stringenten Bedingungen" gemäß der vorliegenden Erfindung wird bei Sambrook et al. (Molecular Cloning, A Laboratory Manual, Cold Spring Harbor Laboratory Press, 1989) definiert. Ein stringente Hybridisierung liegt beispielsweise vor, wenn nach dem Waschen für 1 h mit 1 x SSC und 0,1%SDS bei 50°C, vorzugsweise bei 55°C, besonders bevorzugt bei 62°C und am meisten bevorzugt bei 68°C, insbesondere für 1h in 0,2 x SSC und 0,1 % SDS bei 55°C, vorzugsweise bei 62°C und am meisten bevorzugt bei 68°C noch ein Hybridisierungssignal beobachtet wird. Die Nukleinsäuren, die unter diesen Bedingungen mit der in Seq ID NO 1 gezeigten Nukleinsäure oder einer dieser Sequenz im Rahmen der Degeneration des genetischen Codes entsprechende Nukleotidsequenz hybridisiert, sind auch Gegenstand der vorliegenden Erfindung.

Nukleinsäuren können einzel- oder doppelsträngige DNA, z.B. cDNA, oder RNA, z.B. mRNA, cRNA, pre-mRNA darstellen.

Die in Seq ID NO1 dargestellte Nukleinsäure kodiert für ein humanes ARL-2 Protein.

Bevorzugt ist die Nukleinsäure, die einen Protein kodierenden Abschnitt der in Seq ID NO 1 dargestellten Nukleinsäuresequenz umfaßt. Ein Protein kodierender Abschnitt der in Seq ID NO 1 dargestellten Sequenz liegt im Bereich von Nukleotid 53 bis 1003.
ARL-2 hat 93% Homologie zu ARL-1, dem humanen Homolog von LHOP.

Gegenstand der Erfindung ist ferner eine Nukleinsäure, die für ein Polypeptid mit der in Seq ID NO 2 dargestellten Aminosäuresequenz kodiert.

Die erfindungsgemäße Nukleinsäure ist aus Säugern, z.B. humanen Zellen oder aus einer cDNA-Bibliothek oder einer genomischen Bibliothek, die z.B. aus menschlichen Zellen gewonnen wird, erhältlich. Sie kann nach bekannten Techniken unter Verwendung kurzer Abschnitte der in Seq ID NO 1 gezeigten Nukleinsäureseqenz als Hybridisierungssonden oder Amplifikationsprimer isoliert werden.

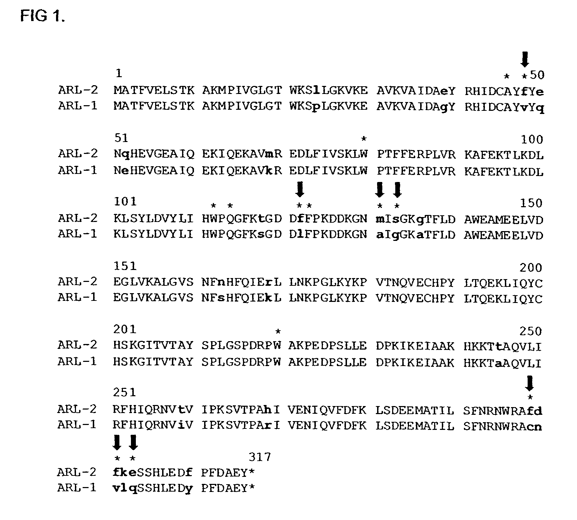

Die Erfindung betrifft weiterhin Polypeptide, die von einer erfindungsgemäßen Nukleinsäure kodiert werden. Diese Polypeptide haben die Funktion eines humanen ARL-2 Proteins und gehören zur Protein-Familie der Aldo-Keto-Reduktasen. Diese Gruppe von Enzymen reduziert Aldehyd- und Ketongruppen verschiedener Substrate. Auch Steroide mit Aldehyd- oder Ketongruppe können zu den Substraten der Aldo-Keto Reduktasen gehören. Die Funktion des ARL-2 Proteins ist die einer Aldo-Keto-Reduktase, die in Zusammenhang steht mit der Apoptose. Substrate des ARL-2 Proteins, wie z.B. 4-Hydroxynonenal, sind cytotoxisch und können Apoptose induzieren. Es konnte gezeigt werden, dass das aliphatische Aldehyd 4-Hydroxynonenal Apoptose in Endothellzellkulturen (Herbst *et al.,* 1997, J Cell Physiol 181, 295-303) und in neuronalen Zellkulturen (Kruman *et al*., 1997, J Neurosci 17, 5089-100,; Malecki *et al*., 2000, J Neurochem 74, 2278-87) induziert. ARL-2 schützt vor Apoptose, indem es die Reduktion dieser toxischen und reaktiven Aldehyde in die weniger reaktive Alkoholform katalysiert. Fehlfunktionen im ARL-2 Protein führen dazu, dass die Zellen Apoptose unterlaufen. Apoptotische Prozesse spielen auch im Ovar, besonders während der Follikulogenese, eine sehr wichtige Rolle. Es ist bekannt, dass ca. 99% der Follikel durch apoptotische Prozesse degenerieren.
Weiterhin Gegenstand der Erfindung ist ein Polypeptid, das die in Seq ID NO 2 dargestellte Aminosäuresequenz umfasst.
Das erfindungsgemäße Polypeptid kann ein rekombinantes Polypeptid, ein natürliches, isoliertes Polypeptid oder ein synthetisches Polypeptid sein.
Das erfindungsgemäße Polypeptid zeigt eine hohe Homologie zu ARL-1, dem humanen LHOP-Homolog, das eine eine wichtige Rolle bei der Ovulation spielt (Brockstedt *et al*., 2000, Endocrinology 141, 2574-2581) und somit für die Regulation der Fertilität von großer Bedeutung ist. In der WO 00/52148 wird erstmals die Sequenz des ARL-1 beschrieben. Ein Maus homologes Protein wurde bereits von Taragnat *et al*., 1988 (J. Reprod. Fert. 83: 835-842) in Samenleitern von männlichen Mäusen beschrieben und als MVDP (**M**ouse **v**as **d**eferens **p**rotein) bezeichnet. Das erfindungsgemäße Polypeptid zeichnet sich durch bestimmte Bereiche im katalytischen Zentrum aus, in denen die Aminosäuresequenz abweicht von der des ARL-1 Proteins. Diese Bereiche spielen nach Jez et al. (Jez et al., 1997, Biochem. Pharmacol., 54, 639 - 647 und Jez und Penning, Chemico-Biol. Interact, 2001, 130-132, 499-525) eine wichtige Rolle bei der Bindung der Substrate im katalytischen Zentrum. (siehe Abbildung 1). Veränderungen der Sequenz in diesen Bereichen tragen zu einer Änderung der Substatspezifität bei, ARL-2 interagiert mit anderen Substraten als ARL-1 und stellt somit ein neues Enzym dar, das eine wichtige Rolle spielt bei der Beseitigung von apoptotischen Substraten. ARL-2 wirkt antiapoptotisch, beim Fehlen bzw. bei Fehlfunktionen von ARL-2 gehen die Zellen in die Apoptose.

Die mRNA des erfindungsgemäßen Polypeptids nach Seq ID NO 2 wird in verschiedenen Geweben transkribiert wie beispielsweise Ovarien, Nebennieren und Vas deferens.

Das erfindungsgemäße Polypeptid oder Teilbereiche davon (Peptide) können zur Herstellung von Antikörpern verwendet werden. Zur Herstellung polyklonaler Antikörper können die Polypeptide oder Peptide z.B. an KLH (Keyhole Limpet Hemocyanin) gebunden werden und Tieren, z.B. Kaninchen, gespritzt werden. Sie können auch zur Herstellung monoklonaler Antikörper verwendet werden. Zur Antikörperherstellung kann ein erfindungsgemäßes Polypeptid oder Peptid oder eine Mischung mehrerer erfindungsgemäßer Peptide verwendet werden. Die Herstellung der Antikörper erfolgt dabei nach Standardverfahren wie sie z.B. in Kohler, G. und Milstein, C., Nature 1975, 256, 495 - 497 und Nelson, P. N. *et al*., Mol. Pathol. 2000, 53, 111 - 117 beschrieben sind.

Gegenstand der Erfindung sind auch die Antikörper, die gegen ein erfindungsgemäßes Polypeptid gerichtet sind.
Die erfindungsgemäßen Antikörper können zur Detektion der erfindungsgemäßen Polypeptide verwendet werden. Dies kann z.B. durch Immunhistochemie erfolgen. Die erfindungsgemäßen Antikörper können auch in anderen Immuntests wie z.B. einem ELISA (enzyme linked immunosorbent assay) oder in Radioimmunotests eingesetzt werden. So kann die Konzentration an erfindungsgemäßen Polypeptid in Gewebe- oder Zellextrakten nachgewiesen werden.

Der Nachweis der Expression des erfindungsgemäßen Polypeptids kann auch über den Nachweis von mRNA in den Zellen erfolgen. Gegenstand der Erfindung ist daher auch die Verwendung einer Sonde mit Nukleinsäuresequenzen, die komplementär zu den Nukleinsäuresequenzen sind, die für die erfindungsgemäßen Peptide kodieren, zur Herstellung eines Reagenz zum Nachweis der Gegenwart von erfindungsgemäßer mRNA in Zellen. Eine Sonde ist ein kurzes DNA-Stück mit mindestens 14 Nukleotiden. Die erfindungsgemäßen Sonden können z.B. in einer Northern Blot Analyse verwendet werden. Diese Methode ist z.B. in Sambrook, J. *et al*., 1989, Cold Spring Harbor Laboratory Press, beschrieben. Andere Methoden zum Nachweis der RNA sind *in situ* Hybridisierung, RNAse Protection Assay oder PCR.

Die Erfindung betrifft außerdem Antisense-Moleküle, die gegen die erfindungsgemäße Nukleinsäuresequenz gerichtet sind und die Expression des ARL-2 Proteins unterdrücken können. Derartige Moleküle können gezielt zur Kontrazeption eingesetzt werden.

Weiterhin sind Gegenstand der Erfindung Vektoren die mindestens eine Kopie der erfindungsgemäßen Nukleinsäure enthalten. Vektoren können prokaryontische oder eukaryontische Vektoren sein. Beispiele für Vektoren sind pPRO (Clontech), pBAD (Invitrogen), pSG5 (Stratagene), pCI (Promega), pIRES (Clontech), pBAC (Clontech), pMET (Invitrogen), pBlueBac (Invitrogen). In diese Vektoren können mit den dem Fachmann bekannten Methoden die erfindungsgemäßen Nukleinsäuren eingefügt werden. Die erfindungsgemäßen Nukleinsäuren befinden sich vorzugsweise in Verbindung mit Expressionssignalen wie z.B. Promotor und Enhancer auf dem Vektor.

Die Erfindung betrifft ferner Zellen, die mit einer erfindungsgemäßen Nukleinsäuresequenz oder mit einem erfindungsgemäßen Vektor transfiziert sind. Als Zellen können z.B. *E. coli,* Hefe, *Pichia,* Sf9, COS, CV-1 oder BHK verwendet werden. Diese Zellen können für die Produktion des erfindungsgemäßen Polypeptids oder für Testsysteme verwendet werden.

Es konnte gezeigt werden, dass LH in der Mitte des Zyklus von der Hypophyse ausgeschüttet wird und die meiotische Reifung der Eizelle sowie die Ovulation der Eizelle bewirkt, und dass ARL-2 unter Kontrolle von LH spezifisch exprimiert wird. Dies lässt darauf schließen, dass Substanzen, die ARL-2 beeinflussen (stimulieren, induzieren oder hemmen) regulativ auf die Eizellreifung, die Follikelreifung, die Steroidogenese und / oder die Ovulation wirken. Eine Hemmung von ARL-2 führt daher zu einer Hemmung der Eizellreifung, der Follikelreifung, der Steroidogenese und / oder der Ovulation. Weiterhin können Substanzen, die ARL-2 beeinflussen (stimulieren, induzieren oder hemmen) auch regulativ auf die Qualität der Eizellen und Follikel wirken. ARL-2 stellt im Vergleich zu dem bereits erwähnten Protein ARL-1 ein neues Enzym mit anderer Substratspezifität dar. Eine Hemmung des ARL-2 führt zur Auslösung von Apoptose. Eine Stimulation des oder eine Verabreichung des beanspruchten rekombinanten Proteins hat den gegenteiligen Effekt, ist antiapoptotisch und fördert die Fertilität (z.B. bei Ovarialinsuffizienz oder beim Auftreten von polycystischen Ovarien). Ein Gegenstand der Erfindung ist deshalb die Verwendung der erfindungsgemäßen Polypeptide oder der dafür kodierenden Nukleinsäuren als Zielsubstanz zur Herstellung eines Mittels zur Behandlung von weiblichen Fertilitätsstörungen, deren Ursache auf einer Fehlfunktion des ARL-2 Proteins basieren.

ARL-2 wird im Vas Deferens exprimiert. Substrate des ARL-2 Proteins, wie das Aldehyd 4-Hydroxynonenal, konnten im humanen Ejakulat identifiziert werden (Gomez *et al*., 1998, Int J Androl 21, 81-94; Selley *et al*., 1991, J Reprod Fertil 92, 291-8) und erhöhte Konzentrationen von 4-Hydroxyalkenalen sind negativ korreliert mit Samen Motilität und Vitalität (Gomez *et al*., 1998, Int J Androl 21, 81-94). Eine Fehlfunktion des ARL-2 Proteins führt zu einer Störung der Reifung der Spermien und zu einer verminderten Spermienqualität und Motilität. Ein Gegenstand der Erfindung ist deshalb die Verwendung der erfindungsgemäßen Polypeptide oder der dafür kodierenden Nukleinsäuren als Zielsubstanz zur Herstellung eines Mittels zur Behandlung von männlichen Fertilitätsstörungen, deren Ursache in einer Fehlfunktion des ARL-2 Proteins liegt.

Insbesondere erfasst die Erfindung die Verwendung
a. einer erfindunggemäßen Nukleinsäure,
b. eines erfindungsgemäßen Polypeptids, oder
c. einer erfindungsgemäßen Zelle
zur Identifizierung von Effektoren eines erfindungsgemäßen Polypeptids. Effektoren sind Substanzen, die auf das erfindungsgemäße Polypeptid inhibitorisch oder aktivierend wirken, und die in der Lage sind, die ARL-2 Funktion der erfindungsgemäßen Polypeptide zu beeinflußen.

Für die gezielte Kontrazeption kann es von Vorteil sein, die Eizellreifung, die Follikelreifung, die Steroidogenese und / oder die Ovulation direkt zu hemmen. Für die gezielte Kontrazeption kann es auch von Vorteil sein, die Spermienreifung direkt zu hemmen und / oder die Qualität der Spermien so zu beeinflussen, dass eine Befruchtung der Eizelle nicht mehr stattfinden kann. Durch Gabe von Antikörpern, die gegen das erfindungsgemäße Polypeptid gerichtet sind, kann die endogene Funktion von ARL-2 beeinträchtigt und / oder aufgehoben werden. Die Erfindung betrifft deshalb auch die Verwendung von Antikörpern die gegen ein erfindungsgemäßes Polypeptid gerichtet sind.

Die Erfindung betrifft weiterhin ein Testsystem zur Identifizierung von Substraten und von Effektoren eines erfindungsgemäßen Polypeptids, wobei ein erfindungsgemäßes Polypeptid als ganzes oder Teilsequenzen davon mit einem Substrat und / oder Modulator inkubiert werden kann und beispielsweise die Reduktion von Ketonen oder Aldehyden gemessen werden kann.

Die Effektoren des erfindungsgemäßen Polypeptids können zur Behandlung von Krankheiten, die auf Fehlfunktionen im ARL-2 Protein beruhen, eingesetzt werden. Beispiele hierfür sind ovarielle Dysfunktion, ,Autoimmune premature ovarian failure', enzündliche Erkrankungen und Erkrankungen des Immunsystems. Außerdem können diese Effektoren zur Behandlung von weiblicher Infertilität eingesetzt werden. Die Effektoren des erfindungsgemäßen Polypeptids können auch zur Behandlung von männlicher Infertilität eingesetzt werden, die auf Fehlfunktionen im ARL-2 Protein beruhen. Beispiel ist verminderte Spermienqualität.
Die Effektoren des erfindungsgemäßen Polypeptids können auch zur Kontrazeption bei der Frau eingesetzt werden. Blockieren sie die Aktivität von ARL-2, kommt es zu einer Hemmung der Eizellreifung, der Follikelreifung, der Steroidogenese und / oder der Ovulation. Die Effektoren können auch zur Kontrazeption beim Mann eingesetzt werden.
Blockieren sie die Aktivität von ARL-2, verhindern sie die Spermienreifung und / oder vermindern die Spermienaktivität, so dass keine Befruchtung der Eizelle mehr stattfinden kann.

Die Erfindung betrifft weiterhin ein Testsystem zur Identifizierung von Effektoren eines erfindungsgemäßen Polypeptids, wobei ein erfindungsgemäßes Polypeptid als ganzes oder Teilsequenzen davon mit einem Modulator inkubiert werden kann. So eignet sich insbesondere ein Enzymassay zum Auffinden von Substanzen, die die Aktivität des erfindungsgemäßen Polypeptids (Reduktion von Aldehyden und Ketonen) stimulieren oder hemmen. Zum Auffinden derartiger Substanzen wird das ARL-2-Protein gereinigt. Dazu kann beispielsweise der kodierende Bereich des ARL-2- Gens in einen Expressionsvektor insertiert werden und das Enzym in pro- oder eukaryontischen Zellen überexprimiert werden (Ausubel *et al*., Current protocols in molecular biology, 1997, Vol. 2; p. 16.0.1). Das so gewonnene rekombinante Protein kann anschließend mit bekannten chromatographischen Methoden, zum Beispiel durch Größenausschlußund/oder Kationen- und/oder Anionenaustauschchromatographie und / oder Affinitätschromatographie aufgereinigt werden und nach Inkubation in Phosphatpuffer mit NAD(P)H als Kofaktor im Enzymtest eingesetzt werden.

Mit Hilfe des beschriebenen Enzymassays können Substanzen ausgetestet werden, die die Reduktion von Substraten der ARL-2- Aldo-Keto Reduktase stimulieren oder hemmen. Die so aufgefundenen stimulierenden Substanzen werden dann zur Herstellung von Mitteln zur Förderung der Fertilität bei der Frau und beim Mann verwendet. Die hemmenden Substanzen werden zur Herstellung von Mitteln, die die Fertilität bei der Frau und/oder beim Mann hemmen, verwendet.

Es wurde gefunden, dass das erfindungsgemäße Polypeptid ein Antigen darstellt, das unbedingt erforderlich ist für die Eizellreifung, die Follikelreifung, die Steroidogenese und / oder die Ovulation. Frauen, die unter Fertilitätsstörungen leiden, könnte direkt das Antigen appliziert werden. Eine Behandlung mit Antikörpern gegen das erfindungsgemäße Polypeptid oder Teilstücken davon kann von Frauen zur Fertilitätskontrolle genutzt werden. Weiterhin wurde gefunden, dass das erfindungsgemäße Polypeptid ein Antigen darstellt, das erforderlich ist für die Spermienreifung und die Sicherung der Spermienqualität. Eine Behandlung mit Antikörpern gegen das erfindungsgemäße Polypeptid oder Teilstücken davon könnte von Männern zur Fertilitätskontrolle genutzt werden.

Weiterhin betrifft die Erfindung ein Verfahren zur Bereitstellung eines pharmazeutischen Mittels, wobei
a. Substanzen mit einem erfindungsgemäßen Testsystem in Kontakt gebracht werden,
b. die Wirkung der Substanzen auf das Testsystem im Vergleich zu Kontrollen gemessen wird,
c. eine Substanz, die in Schritt b. eine Modulation der Aktivität der erfindungsgemäßen Polypeptide zeigt, identifiziert wird
d. und die in Schritt c. identifizierte Substanz mit in der Pharmazie üblichen Formulierungsstoffen gemischt wird.
Unter Aktivität des erfindungsgemäßen Polypeptids ist die Aldo-Keto-Reduktase Aktivität des Polypeptids zu verstehen und die daraus resultierende Schutzfunktion vor Apoptose. Eine Substanz, die durch ein erfindungsgemäßes Verfahren identifiziert wird, kann gegebenenfalls bezüglich metabolischer Stabilität, Aktivität in einem erfindungsgemäßen Testsystem und/oder Bioverfügbarkeit optimiert werden. Dafür können in der Chemie gängige Methoden verwendet werden.
Geeignete pharmazeutische Darreichungsformen enthalten den Wirkstoff in einer Menge, die entweder die Fertilität verhindert oder aber fördert.

Der Wirkstoff kann alleine oder in Kombination mit anderen Wirkstoffen entweder gleichzeitig oder sequentiell verabreicht werden. Die pharmazeutische Zusammensetzung kann neben dem Wirkstoff weitere pharmazeutisch übliche Substanzen enthalten. Die Substanz kann entweder oral, nasal, transdermal, pulmonal oder parenteral, beispielsweise durch Injektion verabreicht werden. Weiterhin denkbar sind Implantate, sowie Vaginalringe oder intrauterine Systeme.

Oral verabreichbare Zusammensetzungen können in Form von Tabletten, Kapseln, Pulvern oder Flüssigkeiten vorliegen. Durch Injektion verabreichbare Zusammensetzungen sind üblicherweise in Form einer parenteral verträglichen wässrigen Lösung oder Suspension. Die bevorzugten Zubereitungen bestehen in einer Darreichungsform, die zur oralen, enteralen oder parenteralen Applikation geeignet ist. Solche Darreichungsformen sind beispielsweise Tabletten, Filmtabletten, Dragees, Pillen, Kapseln, Pulver oder Depotformen sowie Suppositorien. Entsprechende Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmittel wie Carboxypolymethylen, Carboxymethylcellulose, Celluloseacetatphthalat oder Polyvinylacetat, erhalten werden. Die Tabletten können auch aus mehreren Schichten bestehen.

Weiterhin betrifft die Erfindung Verfahren zur Diagnostik von Erkrankungen, deren Ursachen Mutationen des ARL-2 Proteins beinhalten. Hierzu können DNA-Chips verwendet werden. Die Erfindung betrifft daher weiterhin einen DNA―Chip, auf dem mindestens ein Oligonukleotid immobilisiert ist, das der vollständigen cDNA-Sequenz oder einer Teilsequenz bzw. komplementären Sequenz zu der in Seq ID 1 beschriebenen, entspricht. Die Erfindung betrifft somit ferner die Verwendung eines erfindungsgemäßen DNA-Chips zur Diagnose von Fertilitätsstörungen.
DNA-Chips, auch als DNA-Mikroarrays bekannt, sind miniaturisierte Träger, meist aus Glas oder Silizium, auf deren Oberfläche DNA-Moleküle bekannter Sequenz in einem geordneten Raster in hoher Dichte immobilisiert werden. Die Oberflächen-gebundenen DNA-Moleküle werden mit komplementären, eventuell markierten Nukleinsäuren hybridisiert. Die Markierung kann ein Fluoreszenzfarbstoff sein.
Bei Oligonukleotid-Chips stellen die Oligonukleotide, die auf einem erfindungsgemäßen DNA-Chip gebunden sein können, Teilsequenzen der Genprodukte (mRNA bzw. daraus abgeleitete cDNA) dar. Es können ein oder mehrere Oligonukleotide pro Gen auf dem DNA-Chip gebunden sein. Bevorzugt sind 25 Nukleotid-lange Oligonukleotide. Diese werden bevorzugt aus dem jeweiligen 3'untranslatierten Ende des Gens ausgewählt. Methoden zur Herstellung und Verwendung von DNA-Chips sind z.B. in den US-Patenten Nr. 5,578,832; 5,556,752 und 5,510,270 beschrieben.
Bei cDNA-Chips sind die vollständigen Genprodukte (cDNAs) oder Subfragmente (200-500bp lang) auf dem Chip gebunden. Die Methode wird z. B. in Eckmann L *et al*., J. Biol. Chem., 2000, 275(19), 14084-14094 beschrieben.

Zuerst werden die geeigneten DNA-Sequenzen gemäß Seq ID NO 1 bestimmt. Geeignet sind Sequenzen, die mit den ausgewählten Gentranskripten hybridisieren können. Die Oligonukleotide werden dann durch einen chemischen Prozess, der auf photolithographischen Verfahren basiert, auf dem Chip hergestellt. Dazu werden photolithographische Masken verwendet, die durch geeignete Computer-Algorithmen erzeugt wurden.
Die markierte RNA wird mit dem Chip in einem Hybridisierungsofen inkubiert. Anschliessend wird der Chip in einem Scanner analysiert, der die Hybridisierungsprofile bestimmt. Dadurch kann festgestellt werden, ob Veränderungen des Transkripts erfolgt sind (z.B. Mutationen, Trunkierungen). Ebenso ermöglicht es die Quantifizierung des Tranaskripts und so des ARL-2 Proteins und ermöglicht Ruckschlüsse auf z.B. eine Mutation im Promoter.

### Beschreibung der Abbildungen

FIG 1 zeigt einen Sequenzvergleich der ARL-Homologe 1 und 2. Abweichungen in der Aminosäuresequenz der Homologe sind in kleinen Buchstaben angegeben. Die Schlüsselaminosäuren, die eine Rolle bei der Substratbindung spielen (Jez. *et al.),* sind durch Sterne gekennzeichnet. Die Aminosäuren, die eine Rolle bei der Substratbindung spielen und in ARL-1 und ARL-2 verschieden sind, sind durch einen Pfeil gekennzeichnet.

### Beispiele

Die in den Beispielen verwendeten molekularbiologischen Methoden wie z.B. Polymerase-Kettenreaktion (PCR), Herstellung von cDNA, Klonierung von DNA, Sequenzierung von DNA, wurden in bekannten Lehrbüchern wie zum Beispiel in Molecular Cloning, A Laboratory Manual (Sambrook, J. *et al*., 1989, Cold Spring Habor Laboratory Press) beschrieben, durchgeführt.

### Beispiel 1: Klonierung, Expression und Reinigung des humanen ARL-2 Proteins.

Für die Expression des ARL-2-Proteins wurde der kodierende Bereich mittels Polymerasekettenreaktion (Reaktionsbedingungen siehe Bsp2) amplifiziert (Primer für den N-terminalen Bereich: 5' ATG GCC ACG TTT GTG GAG CTC 3'; Primer für den C-terminalen Bereich 5' ATA TTC TGC ATC GAA GGG AAA 3'), in den Baculovirusexpressionsvektor pBlueBac4.5/V5-His-TOPO (Invitrogen) bzw. den eukaryontischen Expressionsvektor pcDNA3.1/V5/His-TOPO (Invitrogen) eingefügt. Für die Expression des ARL-2 Proteins in *E. coli* wurde der kodierende Bereich mittels Polymerasekettenreaktion (Reaktionsbedingungen siehe Bsp2) amplifiziert (Primer für den N-terminalen Bereich: 5' CGG GAT CCG CCA CGT TTG TGG AGC TC 3'; Primer für den C-terminalen Bereich: 5' CCC AAG CTT TCA ATA TTC TGC ATC GAA GGG AAA 3') und in prokaryontischen Expressionsvektor pQE30 (Qiagen) eingefügt. Um Nachweis und Reinigung zu vereinfachen, erfolgte eine Fusion mit einem His-Tag. Nach Cotransfektion von Insektenzellen mit der Bac-N-Blue DNA wurden rekombinante Viren erzeugt, die durch ein PCR-Verfahren identifiziert wurden. Ein Phagenstock wurde dann angelegt und für weitere Transfektionen und Produktion des ARL-2 in größeren Mengen verwendet. In E. coli erfolgte die Expression von ARL-2 nach Inkuktion durch IPTG. Die Reinigung der His-getaggten Proteine erfolgte über eine Nickel- Affinitätssäule nach Angaben des Herstellers (The QIAexpressionist, Qiagen) Die eluierte ARL-2 Protein Fraktion wurde gegen 10 mM Phosphatpuffer dialysiert und im Enzymassay (siehe Bsp. 3) eingesetzt.

### Beispiel 2: Untersuchungen zur Expression der mRNA des humanen ARL-2-Proteins.

Zur Untersuchung der Expression der mRNA wurde eine PCR wie folgt durchgeführt.
Als Template wurde c-DNA von folgenden Geweben verwendet: Knochen, Samenleiter, Prostata, Hoden, Placenta, Brust, Ovar, Nebenniere, Haut, Niere und Lunge. Der Reaktionsansatz enthält: 2 µl cDNA; 20 µM Primer (antisense Primer: 5' TAG ACC TTG GGC CAA ACC TGA G 3'; sense primer: 5'GAG AGG ATT CCT TGA AGT CAA 3'), 10 mM d'NTP; 1,5 mM MgCl₂ und 0,5U Taq Gold (Perkin Elmer). Die PCR-Konditionen sind 94°C 10 min dann 40 Zyklen 94°C 1 min; 58°C 1 min; 72°C 1,5 min. Anschließend wurde ein Aliquot auf ein 1% Agarosegel in 1X TAE-Laufpuffer aufgetragen. Unter diesen Bedingungen konnte eine Expression der mRNA in verschiedenen Geweben gefunden werden (siehe Figur 3).

### Beispiel 3: Enzymassay zum Auffinden von Substanzen, die die ARL-2 Enzymaktivität (Reduktion von Aldehyden und Ketonen) stimulieren.

Ein Standard ARL-2 Enzym-Assay wurde folgendermaßen durchgeführt: Der Standard ENzymreaktionsansatz enzhielt 135 mM Natrium-Phosphatpuffer (pH 6,3), 0,2 mM NADPH und angemessene Substrat- und Enzymkonzentrationen. Die Enzymreaktion erfolgte bei 28°C und der Substratumsatz wurde spektrometrisch über die Abnahme der A₃₄₀ von NADPH bestimmt. Die Reaktion wurde durch die Zugabe von Enzym gestartet. Es wurden standardmäßig Kontrollen ohne Substrat und ohne Enzym gemessen. Mit Hilfe des beschriebenen Enzymasssys können Substanzen getestet werden, die die Reduktion von Substraten des ARL-2 Proteins stimulieren. Die Aldehyde 4-Hydroxynonenal und Isocaproaldehyd zeigten sich als bevorzugte Substrate für die Aldo-Keto Reduktase ARL-2.

### Beispiel 4: Testsystem zum Auffinden von Substanzen, die die ARL-2 Enzymaktivität (Reduktion von Aldehyden und Ketonen) hemmen.

Der oben beschriebene Enzymassay sollte analog wie unter Beispiel 3 beschrieben eingesetzt werden, um Substanzen aufzufinden, die die ARL-2 Aktivität hemmen.

### Beispiel 5: Testsystem zum Auffinden von Apoptose induzierenden Substanzen.

In diesem Testsystem können Substanzen auf ihre Apoptose-induzierende Wirkung hin untersucht werden. Die Adrenocortical Carzinoma Zelllinie Y1 diente als Zellsystem. Zellkulturen dieser Maus Zelllinie wurden für 12 h mit unterschiedlichen Substanzen behandelt, bevor die Apoptoserate in den Zellen bestimmt wurde. 1x10⁻⁵ Y1 Zellen / ml wurden in DME/F12 (+10% FCS) resuspendiert und mit den zu testenden Substanzen behandelt. Nach 12 stündiger Inkubationszeit wurde die Apoptoserate in den yl Zellen über eine Annexin-V-Alexa 568 Färbung (Roche), in Kombination mit einer DNA Färbung (Hoechst-33258, Molecular Probes) nach Angaben des Herstellers bestimmt. Substrate des ARL-2 Proteins, wie das Aldehyd 4-Hydroxynonenal können in einer Konzentration von 10 µM Apoptose in der Y1 Zelllinie induzieren.

### Beispiel 6: Testsystem, in dem die vor Apoptose schützende Funktion von ARL-2 untersucht werden kann.

Das oben beschriebene Testsystem zum Auffinden von Apoptose induzierenden Substanzen in der Y1ZeIllinie (siehe Bsp 5) kann analog verwendet werden um die vor Apoptose schützende Funktion des ARL-2 Proteins zu untersuchen. Substrate des ARL-2 Proteins, wie z.B. 4-Hydroxynonenal, induzieren Apoptose in Y1 Zellen. Die Aldo-Keto Reduktase ARL-2 kann toxische Aldehyde, wie z.B. das 4-Hydroxynonenal in eine weniger bioreaktive Form reduzieren und dadurch Zellen vor Apoptose schützen. Y1 Zellen wurden wie unter Beispiel 5 beschrieben mit den zu untersuchenden Substanzen behandelt; zusätzlich wurde, 12 h vor der Substanzbehandlung, das in einen eukaryontischen Expressionsvektor (pCDNA 3.1 siehe Bsp1) klonierte ARL-2 in die Y1 Zelle mittels LipfectAMINE (Life Technology Inc) nach Angaben des Herstellers transfiziert. 12 h nach Behandlung mit den Substanzen (und folglich 24 h nach der Transfektion) wurde die Apoptoserate in diesen Zellen durch eine Annexin-V Färbung (siehe Bsp 5) bestimmt. Gleichzeitig wurde durch eine ARL-2 Färbung auch die Epxression von ARL-2 untersucht und so die Ko-Lokalisation von ARL-2 und Apoptose in den Zellen untersucht.

## Patentansprüche

1. Nukleinsäure umfassend
a. die in Seq ID NO 1 dargestellte Nukleotidsequenz,
b. eine einer Sequenz aus a. im Rahmen der Degeneration des genetischen Codes entsprechende Nukleotidsequenz oder
c. eine mit den Sequenzen aus a. oder b. unter stringenten Bedingungen hybridisierende Nukleotidsequenz mit der Funktion eines humanen ARL-2-Proteins.

2. Nukleinsäure nach Anspruch 1 umfassend einen Protein-kodierenden Abschnitt der der in Seq ID NO 1 dargestellten Nukleinsäuresequenz.

3. Nukleinsäure, kodierend für ein Polypeptid mit der in Seq ID NO 2 dargestellten Aminosäuresequenz.

4. Polypeptide kodiert von einer Nukleinsäure nach einem der Ansprüche 1-3.

5. Polypeptid, umfassend die in Seq ID NO 2 dargestellte Aminosäuresequenz oder Teile davon.

6. Verwendung eines Polypeptids nach Anspruch 4 und 5 oder von Teilen dieses Polypeptids zur Herstellung von Antikörpern.

7. Antikörper gegen ein Polypeptid nach einem der Ansprüche 4 und 5.

8. Verwendung einer Sonde mit Nukleinsäuresequenzen, die komplementär zu den Nukleinsäuresequenzen nach Anspruch 1-3 sind, zur Herstellung einer Reagenz zum Nachweis der Gegenwart von mRNA und nach einem der Ansprüche 1-3 in Zellen.

9. Antisense-Molekül, das gegen die Nukleinsäure gemäß Anspruch 1 gerichtet ist.

10. Vektor enthaltend mindestens eine Kopie einer Nukleinsäure nach einem der Ansprüche 1-3.

11. Zelle transfiziert mit einer Nukleinsäure nach einem der Ansprüche 1-3 oder mit einem Vektor nach Anspruch 9.

12. Verwendung einer Zelle nach Anspruch 11 zur Expression der Nukleinsäure nach einem der Ansprüche 1-3.

13. Verwendung
a. einer Nukleinsäure nach einem der Ansprüche 1 bis 3,
b. eines Polypeptids nach einem der Ansprüche 4 und 5 oder
c. einer Zelle nach Anspruch 11
zur Identifizierung von Effektoren eines Polypeptids nach Anspruch 4 oder 5.

14. Verwendung einer Nukleinsäure nach Anspruch 1 bis 3 oder eines Polypeptids nach Anspruch 4 und 5 oder des Antikörpers gemäß Anspruch 7 oder eines Antisense-Moleküls nach Anspruch 9 als Zielsubstanz zur Herstellung eines Mittels für Krankheiten, die ursächlich mit dem ARL-2-Gen und / oder Protein zusammenhängen.

15. Testsystem zur Identifizierung von Effektoren eines Polypeptids nach Anspruch 4 oder 5, wobei ein erfindungsgemäßes Polypeptid als ganzes oder Teilsequenzen davon mit einem Modulator (Effektor) inkubiert werden und die Menge an reduzierten Aldehyden und Ketonen gemessen werden.

16. Testsystem nach Anspruch 14 oder 15 wobei die Effektoren die Aldehyd- und Ketonreduzierende Aktivität inhibieren oder aktivieren.

17. Testsystem zur Identifizierung von Effektoren der Enzymaktivität des erfindunggemäßen Polypeptids nach Anspruch 15 in einer erfindungsgemäßen Zelle wobei die Menge an reduzierten Aldehyden und Ketonen gemessen wird.

18. Testsystem zur Identifizierung von Effektoren der vor Apoptose schützenden Funktion des erfindungsgemäßen Polypeptids nach Anspruch 15.

19. Verfahren zur Bereitstellung eines pharmazeutischen Mittels, wobei
a. Substanzen mit einem Testsystem nach Anspruch 15, 16 und 17 in Kontakt gebracht werden.
b. die Wirkung der Substanzen auf das Testsystem im Vergleich zu Kontrollen gemessen wird
c. eine Substanz, die in Schritt b. eine Modulation der Aktivität des Polypeptids gemäß Anspruch 4 oder 5 zeigt, identifiziert wird
d. und die in Schritt c. identifizierte Substanz mit in der Pharmazie üblichen Formulierungsstoffen gemischt wird.

20. DNA-Chip, **dadurch gekennzeichnet**, das mindestens ein Oligonukleotid immobilisiert ist, das der vollständigen cDNA-Sequenz oder einer Teilsequenz bzw. komplementären Sequenz zu der in Seq ID NO 1 beschriebenen, entspricht.

21. Verwendung eines DNA-Chips nach Anspruch 20 zur Diagnose von Fertilitätsstörungen
